(19) Europäisches Patentamt
European Patent Office
Office européen des brevets

(11) Publication number: **0 294 007**
**A1**

# (12) EUROPEAN PATENT APPLICATION

(21) Application number: 88201126.5

(22) Date of filing: 03.06.88

(51) Int. Cl.⁴: C07D 233/40 , C07D 233/42 , C08G 65/34

(30) Priority: 04.06.87 NL 8701308

(43) Date of publication of application: 07.12.88 Bulletin 88/49

(84) Designated Contracting States: AT BE CH DE ES FR GB GR IT LI NL SE

(71) Applicant: STAMICARBON B.V.
Mijnweg 1
NL-6167 AC Geleen(NL)

(72) Inventor: Ramakers, Johannes Arnoldus Hubertus Maria
Gozewijnstraat 17
NL-6191 WV Beek (L.)(NL)

## (54) Urea derivative and a resin composition based thereon.

(57) The invention relates to a urea derivative and a resin composition based on this urea derivative and a polymer with reactive groups.

The urea derivative has the following formula:

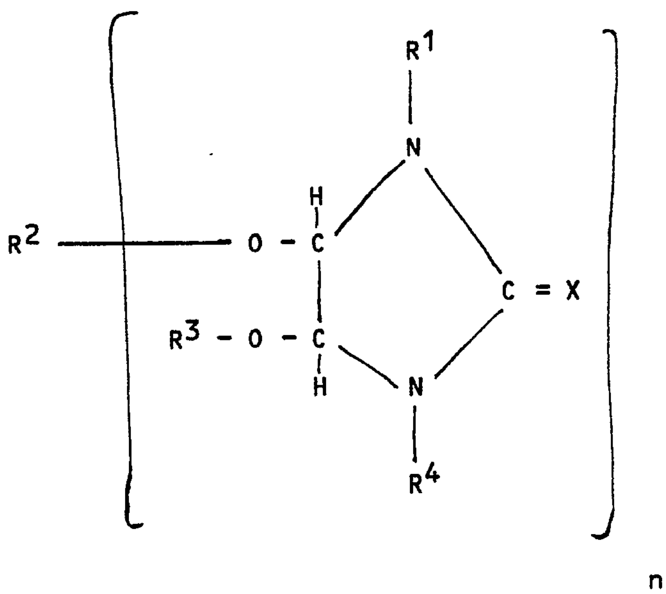

where
X = 0 or S,
$R^1$ = H or $CH_2OR^5$,
$R^2$ = an organic group that is at least m-functional with 3-20 C atoms, and m = 2-20,
$R^3$ = H or $R^2$ or an alkyl group with 1-6 C atoms or an aryl group with 6-10 C atoms,
$R^4$ = H or $CH_2OR^5$,
$R^5$ = H or an alkyl group with 1-6 C atoms,

EP 0 294 007 A1

n = 2-20 and n is less than or equal to m.

## UREA DERIVATIVE AND A RESIN COMPOSITION BASED THEREON

The invention relates to a urea derivative and a resin composition based on this urea derivative and a polymer with reactive groups.

Resin compositions based on a urea derivative and a polymer with reactive groups for application as coating material are described on pages 770-772 of the Encyclopedia of Polymer Science and Engineering (Volume 1, Second Edition, 1985). The urea derivative used there is a cured amino resin. However, cured amino resins are too hard and too brittle to be used as good coatings. When applied in coating mixtures, amino resins are therefore combined with other, more flexible resins with functional groups, such as alkyd resins, polyesters, epoxy resins, acrylate resins, nitrocellulose resins and/or vinyl polymers, to achieve curing or crosslinking. The functional groups of these polymers may be cross-linked with amino resins such as melamine-formaldehyde resins, urea-formaldehyde resins or guanamineformaldehyde resins. Examples of reactive groups in the polymer are carboxyl groups, hydroxyl groups, amide groups, epoxy groups, sulphonic acid groups, phosphate groups and alkoxymethylamide groups. The reactions between, for example, the hydroxyl, carboxyl and/or amide-functional groups and the amino resins may, for example, be effected with an acid catalyst, such as toluenesulphonic acid. The curing reactions may be carried out at room temperature or at increased temperature (up to for example 250¤C). These curing reactions are, however, almost always accompanied by side reactions of N-methylol groups causing formaldehyde emissions which, in view of the current environmental and health requirements, are highly undesirable.

A disadvantage, therefore, of the known resin compositions is that they contain amino resins, which are no longer acceptable as starting material for coatings because, on account of the too high formaldehyde emission, they no longer meet the current environmental requirements applicable to such coatings.

The aim of the invention is to provide a new urea derivative and resin compositions based on a urea derivative and a polymer with reactive groups, with the resin compositions presenting greatly reduced formaldehyde emissions during processing and with the resin compositions resulting in coatings whose formaldehyde emissions meet the current environmental requirements and which, in addition, have the properties required of good coatings.

The resin composition according to the invention is characterized in that it contains a urea derivative with the formula:

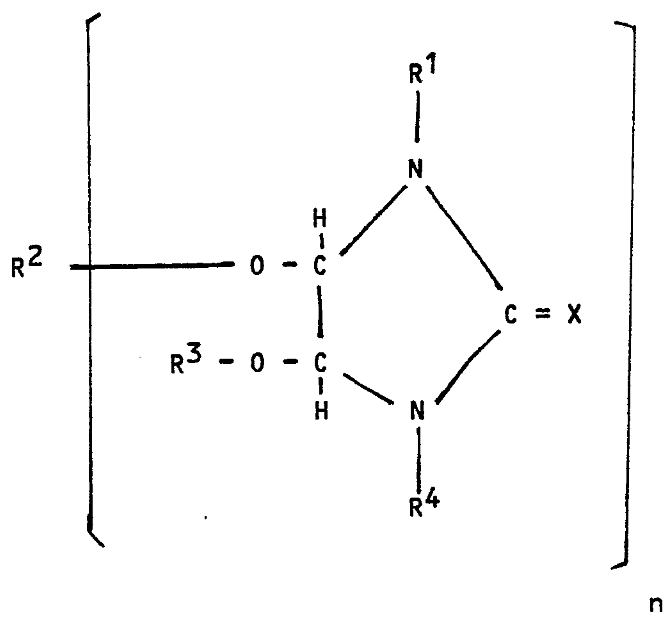

where

X = 0 or S,

$R^1$ = H or $CH_2OR^5$,

$R^2$ = an organic group that is at least m-functional and that contains 3-20 C atoms and m = 2-20,

$R^3$ = H or $R^2$ or an alkyl group with 1-6 C atoms or an aryl group with 6-10 C atoms,

$R^4$ = H or $CH_2OR^5$,
$R^5$ = H or an alkyl group with 1-6 C atoms,
n = 2-20 and n is less than or equal to m.

The invention also relates to the urea derivative as substance.

Preferably an oxygen atom (X = 0) is chosen as X.

According to a preferred embodiment of the invention, the ratio of the total molar amount of $CH_2OR^5$ and the molar amount X is 0.1-2, more in particular 1-2. The urea derivative is in this case methylolated with 0.1-2 molar equivalents of formaldehyde per molar equivalent of ethylene urea derivative, more in particular between 1 and 2 molar equivalents.

Propane-1,2-diol, propane-1,3-diol, butane-1,2-diol, butane-1,4-diol, 2,2-dimethylpropanediol-1,3 (neopentyl glycol), hexane-2,5-diol, hexane-1,6-diol, 2,2-[bis-(4-hydroxy-cyclohexyl)]propane, 1,4-dimethylolcyclohexane, diethyl glycol, dipropylene glycol and 2,2-bis-[4-(2-hydroxyethoxy)phenyl]-propane, glycerol, hexanetriol, pentaerythritol, sorbitol, trimethylolpropane and tris-(2-hydroxyethyl)isocyanurate may, for example, be used as the polyol with 3-20 C-atoms corresponding to the m-functional organic group $R^2$. According to a further preferred embodiment of the invention m is 2,2-8 and n is 2,2-8 and less than or equal to m. Preferably, the m-functional organic group corresponds to tri- or multivalent polyols because the derivatives are capable of creating good crosslinking even at a relatively low degree of methylolation. Preferably, trimethylolpropane, glycerol, sorbitol or mixtures hereof are used as m-functional organic groups.

Depending on the requirements, any $R^1$ and $R^4$ methylol groups and any hydroxy groups present may be, at least partly, etherified with a lower alcohol such as methanol, ethanol, propanol, butanol, isobutanol, pentanol or hexanol. In applications in lacquer systems based on organic solvents butanol is preferably used. Etherification greatly affects the solubility, which increases with the percentage of etherification and with the length of the alkyl radical. In aqueous dispersion lacquer systems the methylol group is preferably applied unetherified.

According to another preferred embodiment of the invention, $R^1$ = $R^4$ = H. This starting compound can be methylolated on the nitrogen atoms. As such, it is unsuitable for binding agents for the coating mixtures described above, but it is easily sellable as intermediate. The non-methylolated compound is usable as such, for example as curing agent in resin mixtures, for example in polymers containing n-methylolacrylamide. In addition, the compound can be used as an additive in, for example, amino resins.

The derivative can be prepared by etherification (condensation) of the functional polyol with 3-20 C atoms with, for example, dihydroxyethylene urea. The molar ratio of the hydroxy groups in the urea compound and the organic group is between 0.1 and 5, preferably between 0.5 and 3, in particular between 0.8 and 2.5. It should be noted that the etherification reaction is usually not completed.

DE-A-3241446, Textile Research Journal (Volume 80, no. 8, August 1970, page 750) and Chemical Abstracts (Volume 73, No. 16, 19 October 1970, 78425 u) describe monomers based on ethylene urea, such as dimethylol dihydroxy ethylene urea (DMDHEU) and dimethylol dihydroxy ethoxy ethylene urea (DMDHeEU). The methylolated reaction products are resulting in coatings with poor properties such as the pendulum hardness, solvent resistance and scrubresistance. In Textile Research Journal a byproduct of the equimolar reaction of ethyleneglycol and DHEU is described. This byproduct is in minor amounts and is not purified. The present invention however is related to a substantially pure product consisting of one or more urea derivatives according to the formula.

US-A-4.442.257 describes dispersions consisting of emulsion polymers with reactive groups and monomeric N-N′-dimethyloldihydroxyethylene urea (DMDHEU), but coatings made from these dispersions present poor properties with respect to, for example, curing, resistance to solvents and scrubresistance.

US-A-4.396.391 describes the reaction products of DMDHEU and a polyol. Unlike the derivatives according to the invention, these reaction products are based on a compound methylolated on the nitrogen atoms, which results in systems that are chemically completely different due to the different reaction sequence. This is also apparent from the process for the etherification of the amino compound, which according to the invention takes place in the melt while the etherifi cation according to the process in US-A-4.396.391 is to be effected in water or a solvent. The resin compositions according to US-A-4396391 are resulting in coatings with poor properties such as the pendulum hardness, solvent resistance and scrubresistance.

According to a preferred embodiment of the invention the resin mixture contains the urea derivative and the polymer with reactive groups in a weight ratio between 90 : 10 and 10 : 90, more in particular between 70 : 30 and 30 : 70.

The urea derivatives according to the invention may be used either separately or combined. In addition, the resin composition according to the invention may contain such an amount of conventional amino resins, such as melamine-formaldehyde resins, urea-formaldehyde resins and/or guanamine-formaldehyde resins,

that it still meets the requirements.

The urea derivative contained by the resin composition according to the invention is preferably a compound based on 4,5-dihydroxyethylene urea and a polyol, more in particular trimethylolpropane or a compound based on 4,5-dialkoxyhydroxyethylene urea and a polyol, more in particular trimethylolpropane. As an example of 4,5-dialkoxyhydroxyethylene urea, 4,5-di($C_1$-$C_4$)-alkoxyhydroxyethylene urea is preferably used.

The resin composition may, for example, be an aqueous dispersion if the polymer with the reactive groups is dispersible in water, or a solution in an organic solvent, for example butanol, special boiling point spirit, butylacetate or xylene, if the polymer with the reactive groups is soluble in an organic solvent, or an aqueous solution, or a combination of these forms.

Curing of the coating material may be effected with common acid catalysts, for example paratoluenesulphonic acid, blocked paratoluenesulphonic acid, naphthalenesulphonic acid, oxalic acid, formic acid or acetic acid.

The polymers with the reactive groups may be anionic, cationic or non-ionogenic polymers. If so desired, the reactive groups may be present separately or simultaneously.

Alkyd resins, saturated and/or unsaturated polyester resins, epoxy resins, polyacrylamides, acrylate resins, vinyl polymers and/or nitrocellulose resins may, for example, be used as polymers with reactive groups.

Examples of suitable alkyd resins may be resins based on polyfunctional alcohols, acids based on natural or synthetic fatty acids and aromatic aliphatic and/or cycloaliphatic polycarboxylic acids or anhydrides with 2-4 carboxyl groups and 3-9 carbon atoms. Usually phthalic acid, maleic acid, fumaric acid, azelaic acid, sebacic acid, trimellitic acid or pyromellitic acid are used as dicarboxylic acids (or anhydrides).

Suitable polyesters may be based on aromatic polycarboxylic acids, for example phthalic acid, isophthalic acid, terephthalic acid, benzene-1,2,4-tricarboxylic acid, pyromellitic acid, trimellitic acid, 3,6-dichlorophthalic acid, tetrachlorophthalic acid or their anhydrides, acid chlorides or lower alkyl esters and on aliphatic diols, such as ethylene glycol, propane-1,2-diol, propane-1,3-diol, butane-1,2-diol, butane-1,4-diol, 2,2-dimethyl-propanediol-1,3 (neopentyl glycol), hexane-2,5-diol, hexane-1,6-diol, 2,2-[bis-(4-hydroxycyclohexyl)]-propane, 1,4-dimethylol-cyclohexane, diethyl glycol, dipropylene glycol and 2,2-bis-[4-(2-hydroxyethoxy)phenyl]-propane and smaller amounts of polyols, for example glycerol, hexanetriol, pentaerythritol, sorbitol, trimethylolpropane and tris-(2-hydroxyethyl)-isocyanurate. Epoxy compounds may also be used instead of diols or polyols. Cycloaliphatic and/or acyclic polycarboxylic acids may also be used as polycarboxylic acids, for example tetrahydrophthalic acid, hexahydroendomethylenetetrahydrophthalic acid, azelaic acid, sebacic acid, decanedicarboxylic acid, dimeric fatty acid, adipic acid, succinic acid, and maleic acid. Hydroxycarboxylic acids and/or, if so desired, lactones may also be used, for example 12-hydroxystearic acid, epsilon-caprolactone or the hydroxypivalic acid ester of neopentyl glycol. Monocarboxylic acids, such as benzoic acid, tert-butylbenzoic acid, hexahydrobenzoic acid and saturated aliphatic monocarboxylic acids may also be added in subordinate amounts. Examples of suitable polyacrylamides are polyacrylamide, polymethacrylamide, polyacrylonitrile and polyalkylacrylamide.

Examples of suitable polymers with reactive carboxyl groups are polymers based on acrylic acid, methacrylic acid, crotonic acid, cinnamic acid, maleic acid, fumaric acid and itaconic acid. The aforementioned monomers may be applied separately or combined and are used with polymerizable monomers containing reactive hydroxy groups or reactive amide groups. They may also be used with monomers containing highly reactive groups (other than the reactive ethylenic double bond with the exception of carboxylic acid groups) such as methyl acrylate, ethyl acrylate, propyl acrylate, ethyl acrylate, propyl acrylate, butyl acrylate, octyl acrylate, decyl acrylate, lauryl acrylate, methyl methacrylate, ethyl methacrylate, butyl methacrylate and heptyl methacrylate. These polymerizable monomers which are free from reactive groups can be used separately or combined for copolymerization with a monomer with a reactive group. Examples of other suitable polymerizable compounds are styrene, o-, m- or p-alkyl styrenes, 2,4-dimethyl styrene, 2,3-dimethyl styrene, 2,5-dimethyl styrene, vinylnaphthalene, methyl vinyl ether, n-butyl vinyl ether, phenyl vinyl ether, acrylonitrile, methacrylonitrile, chlorostyrenes or alkyl styrenes such as $\alpha$-methyl styrene and $\alpha$-ethyl styrene.

Examples of suitable polymers with a reactive hydroxyl group are hydroxyacrylates or methacrylates, for example 2-hydroxyethyl acrylate, 2-hydroxybutyl acrylate, 3-hydroxybutyl acrylate, 4-hydroxybutyl acrylate, 8-hydroxyoctyl acrylate, 2-hydroxyethyl methacrylate, 5-hydroxyhexyl methacrylate, 6-hydroxyoctyl methacrylate, 8-hydroxyoctyl methacrylate, 10-hydroxydecyl methacrylate, 3-hydroxypropyl crotonate, 4-hydroxyamyl crotonate, 5-hydroxyamyl crotonate, 6-hydroxyhexyl crotonate, 7-hydroxyheptyl crotonate and/or 10-hydroxydecyl crotonate.

The resin composition according to the invention may contain common additives, such as flow

improvers, matting agents, emulsifiers, stabilizers, fillers, pigments, thickening agents and/or siccatives.

The resin composition according to the invention can be prepared by mixing the urea derivative, the polymer with the reactive groups and, where applicable, the common additives at a temperature between 0¤C and 100¤C, preferably between 10¤C and 30¤C, at a pressure between $10^4$ Pa and $10^6$ Pa, preferably between $5.10^4$ Pa and $5.10^5$ Pa.

The polymer with the reactive groups can be prepared via all known polymerization methods.

The resin compositions according to the invention can be applied in, for example, coating agents, in the furniture industry, in adhesives, in the automotive industry, in household equipment, in textile applications and in wall paints.

In addition to low formaldehyde emission, the coating compositions according to the invention therefore present good coating properties, for example with respect to curing, pendulum hardness, resistance to solvents, scrubresistance, chemical resistance, brittleness, lustre and scratch resistance.

The environmental requirements to be met with respect to formaldehyde emission are becoming increasingly strict in the various countries. For a number of measuring methods for such emissions, see the Fatipec Proceedings 1982, Liege, Volume II, pages 133-153.

The invention is further elucidated with the following examples, without, however, being limited thereto.

## Example I

### Preparation of a urea derivative according to the invention

#### a) Preparation of 4,5-dihydroxyethylene urea (= DHEU)

This preparation can be effected according to the process described in GB-A-717287. 800 grams of an acid glyoxal solution were neutralized with sodium bicarbonate to a pH of 6.8, after which 500 grams of urea was added a spoonfull at a time while the pH was maintained between 6.5 and 7 with hydrochloric acid.

When the solution obtained had cooled, a first amount of DHEU crystallized, which was subsequently removed by filtration, after which the filtrate was evaporated at 35¤C to about half of its original volume, which caused a second amount of DHEU to crystallize. This was followed by another filtration and the crystals obtained were dried in a vacuum dessicator at 45¤C. The product obtained had a melting point of 137¤C.

#### b) Preparation of a derivative based on dihydroxyethylene urea (DHEU) and trimethylolpropane (TMP)

59.0 grams of DHEU and 44.7 grams of TMP (molar ratio 3 : 2) were mixed at room temperature and subsequently heated to 90¤C in such a manner that a stirrable suspension was obtained.

Then 0.7% phosphoric acid was added as catalyst and the mixture was heated at 90¤C for 30 minutes. The reaction water released was removed in the form of vapour by introduction of nitrogen. After cooling, water was added until a 50% solution was obtained, which was subsequently neutralized to a pH of 7 with sodium hydroxide solution.

GPC analysis revealed that the molecular weights of the products formed varied between 200 and 5000, with peaks at 200, 300 and 500-1500.

#### c) Methylolation of the product obtained in b)

The pH of a 37% formalin solution was set to 8.5 with an 80% sodium hydroxide solution. Then 920 parts by weight of the 50% solution obtained in b) were added dropwise, at a temperature of 30¤C, to 380 parts by weight of the 37% formalin solution, upon which a reaction took place at 40¤C for 30 minutes.

d) Etherification of the product obtained in c)

The aqueous solution was evaporated, after which 350 parts by weight of butanol were added. The water was removed via azeotropic distillation. Butylation took place for 4 hours at 80¤C with concentrated HCl being used as catalyst. After this had been cooled to room temperature, neutralized with 20% NaOH solution and evaporated, the product desired was obtained.

The following examples II and III and the comparative experiments A, B and C describe coating compositions.

Example II

2.691 parts by weight of the product obtained in Ic (with a molar ratio DHEU/TMP of 1.5/1 and fully methylolated, that is, molar ratio F/DHEU = 2.0), 11.433 parts by weight of hydroxyacrylate dispersion (Synresyl TP 868 DF, a hydroxyacrylate copolymer dispersion in water, DSM Resins), 0.860 parts by weight of paratoluenesulphonic acid and 0.017 parts by weight of de-mineralized water were mixed for 5 minutes at room temperature, under atmospheric pressure.

Example III

6.552 parts by weight of the product obtained in Ic (with a molar ratio DHEU/TMP of 1.5/1 and a molar ratio F/DHEU = 1.25), 4.386 parts by weight of hydroxyacrylate dispersion (as in example II), 0.860 parts by weight of paratoluenesulphonic acid, 0.750 parts by weight of ethanol and 2.033 parts by weight of demineralized water were mixed for 5 minutes at room temperature, under atmospheric pressure.

Comparative experiment A

4.168 parts by weight of methylated urea-formaldehyde resin (F/U molar ratio of 2.43), 9.141 parts by weight of hydroxyacrylate dispersion (as in example II), 0.220 parts by weight of paratoluenesulphonic acid, 0.457 parts by weight of ethanol and 1.005 parts by weight of demineralized water were mixed for 5 minutes at room temperature, under atmospheric pressure.

Comparative experiment B

4.168 parts by weight of methylated urea-formaldehyde resin (F/U molar ratio 2.21), 8.761 parts by weight of hydroxyacrylate dispersion (as in example II), 0.220 parts by weight of paratoluenesulphonic acid, 0.457 parts by weight of ethanol, 1.028 parts by weight of demineralized water and 0.336 parts by weight of urea were mixed for 5 minutes at room temperature, under atmospheric pressure.

Comparative experiment C

3.410 parts by weight of N,N′-dimethyloldihydroxyethylene urea (DMDHEU, a monomer with F/DMEU ratio of 2.0), 10.703 parts by weight of hydroxyacrylate dispersion (as in example II), 0.845 parts by weight of paratoluenesulphonic acid and 0.044 parts by weight of demineralized water were mixed for 5 minutes at room temperature, under atmospheric pressure.

The coating mixtures according to examples II and III and comparative experiments A, B and C were applied to a glass plate, in wet condition, with a layer thickness of 100 um, and then tested to determine the formaldehyde emission and coating properties.

The formaldehyde emission (see Table 1) was determined by means of the 'ventilated interstice system' (described in the Fatipec Proceedings 1982, Liege, Volume II, page 140-153).

The curing behaviour of the coating was evaluated by means of the pendulum hardness test according to Koenig (DIN 53157) (Table 2).

Table 1 shows that the formaldehyde emission ( = F-EM) of the coatings according to examples II and III is much lower than that of the coatings according to the comparative experiments A and B and after 240

minutes is also much lower than that according to comparative experiment C.

Table 2 shows that with respect to the pendulum hardness according to Koenig (high values are aimed at), the coatings according to examples II and III and according to the comparative experiments A and B present good pendulum hardness, whereas the coating according to comparative experiment C presents an unacceptable value.

Consequently, the formaldehyde emission as well as the pendulum hardness of the coatings according to examples II and III are good, whereas the coatings according to the comparative experiments A and B present poor formaldehyde emissions and the coatings according to comparative experiment C present unacceptably poor pendulum hardness.

TABLE 1

| COATING ACCOR- DING TO: | TIME PEAK (MIN) | F-EM. PIEK ($mg/m^3$) | F-EM. 60 min ($mg/m^3$) | F-EM. 120 min ($mg/m^3$) | F-EM. 240 min ($mg/m^3$) | F-EM. 360 min ($mg/m^3$) | F-EM. 24 hr ($mg/m^3$) | F-EM. 1 week ($mg/m^3$) | F-EM. 2 wek. ($mg/m^3$) | F-EM. 4 wek. ($mg/m^3$) |
|---|---|---|---|---|---|---|---|---|---|---|
| Ex. II | 30 | 48.5 | 46.6 | 39.0 | 2.2 | 1.3 | 0.193 | 0.115 | 0,139 | 0,063 |
| Ex. III | 6 | 12.2 | 9.2 | 0.80 | 0.50 | 0.45 | 0.11 | X | X | X |
| Exp. A | 85 | 167.0 | 142.0 | 155.0 | 14.0 | 8.0 | 5.3 | 1.44 | 1,068 | 0,363 |
| Exp. B | 100 | 117.8 | 90.7 | 106.3 | 6.9 | 6.7 | 6.3 | 1.40 | 0,569 | 0,304 |
| Exp. C | 17.5 | 31.9 | 26.0 | 21.4 | 20.5 | 7.8 | 1.9 | XX | XX | XX |

X = emission was so low that it could no longer be determined

XX = not determined

TABLE 2

| COATING ACCORDING TO: | PENDULUM HARDNESS IN SECONDS 1 H | 2 H | 4 H | 6 H | 1 DAY | 2 DAYS | 1 WEEK | 2 WEEKS |
|---|---|---|---|---|---|---|---|---|
| Ex. II | 30 | 76 | 135 | 152 | 159 | 168 | 182 | 175 |
| Ex. III | 7 | 22 | 74 | 99 | 166 | 175 | 142 | 157 |
| Exp. A | 21 | 33 | 54 | 67 | 100 | 146 | 198 | 210 |
| Exp. B | - | 21 | 35 | 37 | 106 | 142 | 180 | 182 |
| Exp. C | 18 | 25 | 26 | 28 | 42 | 50 | - | - |

TABLE 3

Qualitative summary of coating properties

| Coating according to: | dryness | pot life | pendulum hardness |
|---|---|---|---|
| Example II | 1 - 2 | 1 | 1 - 2 |
| Example III | 1 | 1 | 2 |
| Exp. A | 1 - 2 | 4 | 1 |
| Exp. B | 3 | 4 | 1 - 2 |
| Exp. C | 1 - 2 | 1 | 5 |

where:
1 = very good
2 = good
3 = reasonable
4 = insufficient
5 = unacceptable

The 'dryness' was determined by pressing a piece of cotton wool against the coating for 10 seconds using a 1 kg weight and then visually determining whether the cotton wool still adhered to the coating.

The 'pot life' was determined visually with the moment at which gelling becomes visible being applied as criterion.

The 'pendulum hardness' was determined according to Koenig's test method (DIN 53157).

## Claims

1. Urea derivative with the formula:

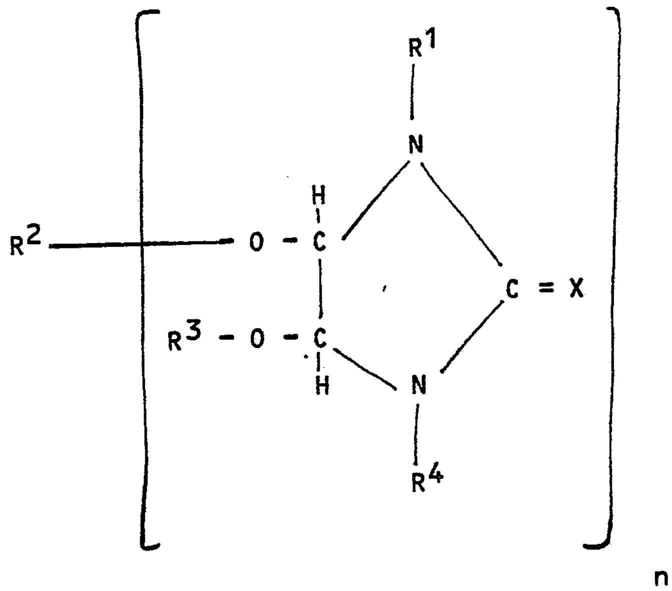

where
$X = 0$ or S,
$R^1 = H$ or $CH_2OR^5$,
$R^2$ = an organic group that is at least m-functional with 3-20 C atoms and $m = 2\text{-}20$,
$R^3 = H$ or $R^2$ or an alkyl group with 1-6 C atoms or an aryl group with 6-10 C atoms,
$R^4 = H$ or $CH_2OR^5$,
$R^5 = H$ or an alkyl group with 1-6 C atoms,
$n = 2\text{-}20$ and n is less than or equal to m.

2. Urea derivative according to claim 1, characterized in that $X = 0$.

3. Urea derivative according to claim 1 or 2, characterized in that the ratio of the total molar amount of $CH_2OR_5$ and the molar amount of X is 0.1-2.

4. Urea derivative according to any one of claims 1-3, characterized in that the m-functional organic group corresponds to a tri- or higher-functional polyol.

5. Urea derivative according to any one of claims 1, 2 and 4, characterized in that $R^1 = R^4 = H$.

6. Resin composition based on an urea derivative and a polymer with reactive groups, characterized in that the resin composition contains an urea derivative according to any one of claims 1-4.

7. Resin composition according to claim 4, characterized in that the resin composition contains the urea derivative and the polymer with reactive groups in a weight ratio between 90 : 10 and 10 : 90.

8. Resin composition according to any one of claims 6-7, characterized in that the urea derivative contained by the resin composition is a compound based on 4,5-dihydroxyethylene urea and a polyol.

9. Resin composition according to any one of claims 6-7, characterized in that the urea derivative contained by the resin composition is a compound based on 4,5-dialkoxyethylene urea and a polyol.

10. Coating composition obtained with the resin composition according to any one of claims 6-9.

European Patent Office

# EUROPEAN SEARCH REPORT

Application Number

EP 88 20 1126

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | TEXTILE RESEARCH JOURNAL, vol. 40, no. 8, 1st August 1970, pages 749-760, Textile Research Institute, Princeton, NJ, US; C.V. STEVENS et al.: "Cross-linking of cotton cellulose with ethylene urea derivatives having varying hydrogen-bonding capabilities: part I: Effects on the physical properties and the hydrogen-bonded structure" * Page 75 * |  | C 07 D 233/40<br>C 07 D 233/42<br>C 08 G 65/34 |
| A | CHEMICAL ABSTRACTS, vol. 73, no. 16, 19th October 1970, page 57, abstract no. 78425u, Columbus, Ohio, US; C.V. STEVENS et al.: "Crosslinking cotton cellulose with ethyleneures derivatives having varying hydrogen-bonding capabilities. II. Accessibility determination", & J. APPL. POLYM. SCI. 1970, 14(7), 1691-700 * Abstract * |  | TECHNICAL FIELDS SEARCHED (Int. Cl.4) |
| A | DE-A-3 241 446 (BASF) * Whole document * |  | C 07 D 233/00<br>C 08 G 65/00 |
| D,A | US-A-4 396 391 (SUN CHEMICAL CORP.) * Whole document * |  |  |

The present search report has been drawn up for all claims

| Place of search | Date of completion of the search | Examiner |
|---|---|---|
| THE HAGUE | 07-09-1988 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document